# EUROPEAN PATENT APPLICATION

(11) **EP 4 641 205 A1**
(43) Date of publication of application: **29.10.2025**
(21) Application number: 23907848.8
(22) Date of filing: 22.12.2023
(51) Int. Cl.: G01N 33/68

(54) **METHOD FOR SELECTING STEM CELLS WITH ENHANCED CARTILAGE DIFFERENTIATION CAPABILITY AND CELL THERAPY PRODUCT COMPRISING SAME**

(30) Priority: 23.12.2022 KR 20220182828
(71) Applicant: Kangstem Biotech Co., Ltd., Seoul 06179 (KR)
(72) Inventor: KIM, Mi Jin, Seoul 08590 (KR); AHN, Jong Chan, Bucheon-si, Gyeonggi-do 14597 (KR); SONG, Seong Soo, Anyang-si, Gyeonggi-do 13934 (KR); LEE, Seung Hee, Seoul 08797 (KR); KANG, Kyung Sun, Seoul 06338 (KR)
(74) Representative: Regimbeau
(86) International application number: PCT/KR2023/021362
(87) International publication number: WO 2024/136565

(57) **Abstract**

The present invention pertains to a method for selecting stem cells with enhanced cartilage differentiation capability, a cell therapy product comprising the stem cells selected by the selection method, and a production method therefor.

## Description

### [Technical Field]

The present invention relates to a method for selecting stem cells with enhanced cartilage differentiation capability, a cell therapy product containing stem cells selected by the selection method, and a method for manufacturing the cell therapy product.

### [Background Art]

A joint is a site where bones come together and is composed of cartilages, joint capsules, synovial membranes, ligaments, tendons, muscles, and others. Articular cartilage is easily injured and prone to pathological degeneration. Once damaged, articular cartilage generally does not heal or only partially heals under specific biological conditions.

Arthritis is an inflammation of joints caused by various factors, and is often accompanied by fever, pain, stiffness, and swelling. There are more than 100 causes of arthritis, such as degenerative changes, immune system disorders, infections, trauma, and metabolic disorders.

As for existing treatments for arthritis, there are no effective medications, and, non-medicinal treatments currently predominate over medicinal treatments. The medications include NSAID-based pain-relieving and anti-inflammatory agents for mitigating symptoms, such as inflammation and pain, steroid preparations, glucosamine, and the like, and treatments using hyaluronic acid or the like are mainly employed to protect joints through a lubrication action by intra-articular injections.

Surgical therapy, such as artificial joint replacements corresponding to non-medicinal treatments, is performed for severe arthritis, but this requires rehabilitation, has a limited replacement joint lifespan (within 10 years), incurs costs, and causes side effects, such as inflammation resulting from implant corrosion, and secondary infections. Therefore, in a clinical aspect, a therapy of delaying surgery as long as possible is required, and considering the efficacy and side effects of existing treatments, there is a need for the development of new medicines that are safe and effective in preventing joint wear. Cell therapy is being illuminated as a promising tissue generation method, but quality issues to obtain satisfactory solutions need to be addressed.

### [Prior Art Document]

(Patent Document 1) Korean Patent No. 10-1669423

### [Disclosure]

### [Technical Problem]

The present invention is directed to a method for selecting stem cells with enhanced cartilage differentiation capability, a cell therapy product containing stem cells selected by the selection method, and a method for manufacturing the cell therapy product.

### [Technical Solution]

An aspect of the present invention is to provide a method for manufacturing a cell therapy product.

Another aspect of the present invention is to provide a pharmaceutical composition for the prevention or treatment of arthritis.

Still another aspect of the present invention is to provide a method for selecting stem cells for the prevention or treatment of arthritis.

### [Advantageous Effects]

The methods of the present invention can produce a cell therapy product with enhanced efficacy by effectively selecting stem cells with enhanced cartilage differentiation capability.

### [Brief Description of Drawings]

FIG. 1 shows the results of analyzing the concentration of TGF-β1 secreted per 1x10⁵ cells in umbilical cord blood-derived mesenchymal stem cells of eight lots at passage P2.
FIG. 2 shows the results of identifying the cartilage differentiation capability of umbilical cord blood-derived mesenchymal stem cells of eight lots at passage P6 through Safranin-O staining.
FIG. 3 shows the results of identifying the cartilage differentiation capability of umbilical cord blood-derived mesenchymal stem cells of eight lots at passage P6 through Safranin-O staining and quantitative evaluation using a program.
FIG. 4 shows the results of analyzing correlation coefficients between TGF-β1 secretion ability result values at passage P2 and Safranin O positive area (%) result values at passage P6 in the umbilical cord blood-derived mesenchymal stem cells of eight lots.
FIG. 5 shows the results of evaluating cartilage tissue regeneration capability after *in vivo* administration for each of High and Low groups classified by TGF-β1 secretion capability of stem cells at passage P2.

### [Detailed Description of the Invention]

An aspect of the present invention is directed to a method for manufacturing a cell therapy product.

In one embodiment, the method for manufacturing a cell therapy product may include: measuring the concentration of TGF-β1 within a culture of stem cells subcultured for 2 passages or fewer; and isolating stem cells secreting TGF-β1, the concentration of which exceeds a specific value within the culture.

In the method for manufacturing a cell therapy product according to the previous embodiment, the method may include measuring the concentration of TGF-β1 within a culture of stem cells subcultured for 2 passages or fewer.

In the method for manufacturing a cell therapy product according to any one of the previous embodiments, the method may further include culturing the stem cells secreting TGF-β1, the concentration of which exceeds a specific value within the culture.

In the method for manufacturing a cell therapy product according to any one of the previous embodiments, the method is characterized by predicting the cartilage differentiation capability of stem cells at passage 6 or higher through the assay of TGF-β1 secretion ability at passage 2 or lower corresponding to an early passage.

In the method for manufacturing a cell therapy product according to any one of the previous embodiments, the method may include culturing the stem cells secreting TGF-β1, the concentration of which exceeds a specific value within the culture, and proliferating the stem cells.

In the method for manufacturing a cell therapy product according to any one of the previous embodiments, the method may include additionally subculturing the stem cells secreting TGF-β1, the concentration of which exceeds a specific value within a culture of stem cells subcultured for 2 passages or fewer.

In the method for manufacturing a cell therapy product according to any one of the previous embodiments, the method may include culturing the stem cells secreting TGF-β1, the concentration of which exceeds a specific value within the culture, and proliferating and differentiating the stem cells.

In the method for manufacturing a cell therapy product according to any one of the previous embodiments, the cell therapy product may contain stem cells secreting TGF-β1, the concentration of which exceeds a specific value within a culture of stem cells subcultured for 2 passages or fewer, or cells differentiated or proliferated from the stem cells.

In the method for manufacturing a cell therapy product according to any one of the previous embodiments, the method may include isolating stem cells secreting TGF-β1, the concentration of which exceeds 1046.7 pg/1x10⁵ cells within a culture of stem cells subcultured for 2 passages or fewer.

In the method for manufacturing a cell therapy product according to any one of the previous embodiments, the method may include isolating stem cells secreting TGF-β1, the concentration of which is no less than 1132.12 pg/1x10⁵ cells within a culture of stem cells subcultured for 2 passages or fewer.

In the method for manufacturing a cell therapy product according to any one of the previous embodiments, the method may include isolating stem cells secreting TGF-β1, the concentration of which is no less than 1486.7 pg/1x10⁵ cells within a culture of stem cells subcultured for 2 passages or fewer.

In the method for manufacturing a cell therapy product according to any one of the previous embodiments, the stem cells may be umbilical cord blood-derived mesenchymal stem cells.

In the method for manufacturing a cell therapy product according to any one of the previous embodiments, the method may include: isolating stem cells secreting TGF-β1, the concentration of which exceeds 1046.7 pg/1x10⁵ cells within a culture of stem cells subcultured for 2 passages or fewer; and proliferating and differentiating the stem cells.

In the method for manufacturing a cell therapy product according to any one of the previous embodiments, the method may include isolating stem cells secreting TGF-β1, the concentration of which exceeds 1046.7 pg/1x10⁵ cells within a culture of stem cells subcultured for 2 passages or fewer to select the stem cells as an active ingredient of the cell therapy product.

In the method for manufacturing a cell therapy product according to any one of the previous embodiments, the stem cells may secrete TGF-β1, the concentration of which exceeds 1046.7 pg/1x10⁵ cells within the culture at the time of subculture for 2 passages or fewer.

In the method for manufacturing a cell therapy product according to any one of the previous embodiments, the stem cells may secrete TGF-β1, the concentration of which exceeds 1046.7 pg/1x10⁵ cells within the culture of stem cells at the time of subculture for 2 passages or fewer.

In the method for manufacturing a cell therapy product according to any one of the previous embodiments, the cell therapy product contains stem cells or differentiated cells, which are proliferated or differentiated from stem cells secreting TGF-β1, the concentration of which is no less than 1132.12 pg/1x10⁵ cells within the culture of stem cells at the time of subculture for 2 passages or fewer.

In the method for manufacturing a cell therapy product according to any one of the previous embodiments, the cell therapy product contains stem cells or differentiated cells, which are proliferated or differentiated from stem cells secreting TGF-β1, the concentration of which is no less than 1486.7 pg/1x10⁵ cells within the culture of stem cells at the time of subculture for 2 passages or fewer.

In the method for manufacturing a cell therapy product according to any one of the previous embodiments, the stem cells secreting TGF-β1, the concentration of which exceeds a specific value within the culture, may exhibit excellent cartilage differentiation capability compared with stem cells secreting TGF-β1, the concentration of which is no more than a specific value within the culture. In the method for manufacturing a cell therapy product according to any one of the previous embodiments, the cell therapy product is a cell therapy product for the prevention or treatment of arthritis.

In the method for manufacturing a cell therapy product according to any one of the previous embodiments, the method may include differentiating the isolated stem cells into cartilage cells.

Another aspect of the present invention is directed to a pharmaceutical composition for the prevention or treatment of arthritis, the pharmaceutical composition containing, as an active ingredient, stem cells or cells differentiated therefrom.

In one embodiment, the cells contained in the pharmaceutical composition may be proliferated or differentiated from stem cells secreting TGF-β1, the concentration of which exceeds a specific value within a culture.

In the pharmaceutical composition according to the previous embodiment, the cells contained in the pharmaceutical composition may be proliferated or differentiated from stem cells secreting TGF-β1, the concentration of which exceeds a specific value within a culture of stem cells at the time of subculture for 2 passages or fewer.

In the pharmaceutical composition according to any one of the previous embodiments, the cells contained in the pharmaceutical composition may be proliferated or differentiated from stem cells secreting TGF-β1, the concentration of which exceeds 1046.7 pg/1x10⁵ cells within a culture of stem cells at the time of subculture for 2 passages or fewer.

Another aspect of the present invention is directed to a method for selecting stem cells suitable for the prevention or treatment of arthritis.

Another aspect of the present invention is directed to a method for selecting stem cells for the prevention or treatment of arthritis.

In one embodiment, the method may include: measuring the concentration of TGF-β1 within a culture of stem cells; measuring the concentration of TGF-β1 within a culture of stem cells; and isolating, as stem cells for the prevention or treatment of arthritis, stem cells secreting TGF-β1, the concentration of which exceeds a specific value within the culture.

In the method for selecting stem cells for the prevention or treatment of arthritis according to the previous embodiment, the method may include selecting stem cells secreting TGF-β1, the concentration of which exceeds 1046.7 pg/1x10⁵ cells within the culture.

The present invention will be specifically described as follows. Each description and exemplary embodiment disclosed herein may also be applied to other descriptions and exemplary embodiments. That is, all combinations of various elements disclosed herein fall within the scope of the present invention. Furthermore, the scope of the present invention is not limited by the specific description below.

Furthermore, a person skilled in the art will recognize or be able to ascertain many equivalents to the specific embodiments of the present invention described herein, by using no more than routine experimentation. Furthermore, these equivalents are intended to be included in the present invention.

Furthermore, throughout the overall specification, many papers and patent documents are referenced and their citations are provided. The disclosures of cited papers and patent documents are entirely incorporated by reference herein, and the level of the technical field within which the present invention falls and details of the present invention are explained more clearly.

The term "cell therapy product" as used herein refers to cells and tissues that have been isolated and cultured from a subject and prepared through special manipulation and are used as pharmaceutical products for the purpose of treatment, diagnosis, and prevention. The term means a pharmaceutical product for use in treating, diagnosing, and preventing diseases through a series of actions including proliferating and selecting living autologous cells, allogeneic cells, or xenogeneic cells in vitro or changing the biological characteristics of cells in other ways for the purpose of restoring the functions of cells or tissues.

The cell therapy product of the present invention may be used without freeze-drying or freeze-dried for later use. When the freeze-drying is required, standard cryo-preservatives (e.g., DMSO, glycerol, and Epilife cell freeze-drying medium (Cascade Biologics)) may be added to a cell population before freeze-drying. Additionally, the cell therapy product may be administered after being formulated into a unit administration form suitable for administration into a patient's body according to the conventional method in the pharmaceutical field, and the formulation may include a dose that is effective after a single or several administrations. Examples of the formulations suitable for this purpose, as a formulation for parenteral administration, may preferably be injections such as injection ampoules, infusion agents such as infusion bags, and spraying agents such as aerosol formulations. The injection ampoules may be prepared by mixing with an injection immediately prior to use. For injection solutions, physiological saline, glucose, mannitol, Ringer's solution, or the like may be used. Additionally, for infusion bags, materials of polyvinyl chloride or polyethylene may be used, and infusion bags manufactured by Baxter, Becton Dickinson, Medcep, National Hospital Products, or Terumo may be exemplified.

The cells used for the cell therapy product may be stem cells or cells differentiated from stem cells.

In an embodiment, the cells contained in the cell therapy product of the present invention may be stem cells secreting TGF-β1, the concentration of which exceeds 1046.7 pg/1x10⁵ cells within a culture of stem cells subcultured at the time of subculture for 2 passages or fewer, or stem cells or differentiated cells, which are proliferated or differentiated therefrom.

In an embodiment, the cells contained in the cell therapy product of the present invention may be stem cells secreting TGF-β1, the concentration of which exceeds 1046.7 pg/1x10⁵ cells within a culture of stem cells at passage 2 (P2), or stem cells or differentiated cells, which are proliferated or differentiated therefrom.

In an embodiment, the cells contained in the cell therapy product of the present invention may be stem cells obtained by additionally subculturing stem cells secreting TGF-β1, the concentration of which exceeds 1046.7 pg/1x10⁵ cells within a culture at P2 (passage 2), to expand the stem cells to P6 (passage 6), or stem cells or cells, which are differentiated therefrom.

In an embodiment, the concentration of TGF-β1 may exceed 1046.7 pg/1x10⁵ cells or exceed 1109.49 pg/1x10⁵ cells, or may be no less than 1132.12 pg/1x10⁵ cells, no less than 1146.8 pg/1x10⁵ cells, no less than 1450.57 pg/1x10⁵ cells, or no less than 1486.7 pg/1x10⁵ cells.

The cell therapy product of the present invention may be administered by the conventional administration method used in the art along with other stem cells, which are used for transplantation and other uses, or in a mixture form with such stem cells, and may be preferably engrafted or transplanted directly to the disease area, or directly transplanted or infused to the abdominal cavity of a patient in need of treatment, but is not limited thereto. Additionally, the administration may be conducted either by a non-surgical administration method using a catheter or by a surgical administration method, such as injecting or transplanting after the incision of the disease area. Furthermore, the cell therapy product may also be administered parenterally, for example, intravenous injection, which is one of the general methods for transplantation of stem cells of hematopoietic system, besides direct administration to the lesion.

The cell therapy product may be administered in a single dose or in divided doses. However, it should be understood that the actual dose of an active ingredient is determined considering various related factors, such as disease to be treated, severity of disease, administration route, and body weight, age, and sex of a patient, and thus the dose should not be construed as limiting the scope of the present invention in any aspect.

The term "stem cells" as used herein refers to cells having the potential to differentiate into various tissues, i.e., an undifferentiated cell. The stem cells may be derived from humans or animals, or may be derived from the umbilical cord, umbilical cord blood, bone marrow, fat, muscle, nerve, skin, amnion, or placenta. As an example, the stem cells may be mesenchymal stem cells.

The term "mesenchymal stem cells" as used herein refers to a heterogeneous population of stem cells having self-renewal and the potential to differentiate into mesodermal lineage and different embryonic lineages such as endodermal and ectodermal lineages. The mesenchymal stem cells may be used interchangeably with the multipotent undifferentiated cells, and may be adult stem cells with the potential to differentiate into various mesodermal cells, such as adipocytes, osteoblasts, chondrocytes, cardiomyocytes, or myocytes, or also ectodermal cells, such as neuronal cells.

Specifically, the stem cells of the present invention may be umbilical cord blood-derived mesenchymal stem cells.

The term "subculture" as used herein is one of the cell proliferation methods, and refers to a culture method of transferring cells from a previous culture to a fresh medium periodically, for example, every 1 to 7 days. The subculture is a culture method that preserves cell lines and maintains cell generations, and this method is called subculturing or passaging. Such subculture allows for the removal of accumulated toxic metabolites and the replenishment of depleted nutrients, thereby preventing cell death and promoting growth and proliferation. The subculture may be performed when the cells cover approximately 70% to 90% of the culture surface area. The term "passage 2" as used herein refers to a state in which cells have undergone two rounds of subculturing for proliferation.

The term "differentiation" as used herein refers to a process by which during the division, proliferation, and growth of cells, the cells become specialized in structure and function, that is, the cells, tissues, and the like of organisms are transformed in terms of morphology or function to perform the given roles thereof. For example, a state in which, in ontogeny, qualitative differences are made among parts of any biological system that were initially homologous, or as a result thereof, the system is divided into qualitatively distinguishable partial systems is referred to as differentiation.

The term "differentiated cells" refers to any cells that are at the stage of differentiating into somatic cell lineages or have finally differentiated. In other words, the differentiated cells mean cells that constitute an adult and have restricted differentiation potential and self-renewal.

For the stem cell culture of the present invention, a medium known for stem cell culturing may be used, and specifically, KSB-3 medium may be used.

In an embodiment of the present invention, as a result of measuring the concentration of TGF-β1 within a culture obtained by culturing cord blood-derived stem cells in KSB-3 complete media to P2, a group with a concentration of TGF-β1 within the culture exceeding 1046.7 pg/1x10⁵ cells showed the significantly high capability to differentiate into cartilage cells, but a group with a concentration of the above value or lower showed the low capability to differentiate into cartilage cells. Therefore, the present invention was completed by providing the value as a criterion for selecting cells that can be used as a cell therapy product due to excellent differentiation capability.

The term "arthritis" as used herein may refer to chronic inflammation of tissues around joints, such as tendons, ligaments, and muscles, as well as joints in other organs. The arthritis may be at least one selected from the group consisting of osteoarthritis, osteochondritis dissecans, ligament injuries of the joint, meniscal injuries, infectious arthritis, psoriatic arthritis, ankylosing spondylitis, rheumatoid arthritis, and juvenile rheumatoid arthritis.

Osteoarthritis, also referred to as degenerative arthritis, is a type of arthritis, which is caused by degenerative changes in the cartilage and surrounding bones of synovial joints. Osteoarthritis may be caused due to cartilage damage caused by aging or excessive physical stress (e.g., obesity, trauma), and is characterized by a progressive loss of articular cartilage, bone hypertrophy beneath the cartilage, bone formation at the edge of the joint, and non-specific synovial inflammation.

Rheumatoid arthritis is a chronic autoimmune disease characterized by inflammation and proliferation of synovial cells, resulting in osteoporosis and bone erosion in the bones surrounding the joints. When rheumatoid arthritis progresses to some degree, the gradual destruction of joint cartilage results in narrowing of the joint space and loss of tension in the joint capsule and ligaments.

The term "prevention" as used herein encompasses any action that inhibits or delays the occurrence of a disease.

The term "treatment" as used herein encompasses any action that alleviates or advantageously changes the symptoms of a disease.

The pharmaceutical composition of the present invention may further contain a pharmaceutically acceptable salt.

The term "pharmaceutically acceptable carrier" refers to a carrier or a diluent that does not inhibit biological activity and characteristics of an injected compound while causing no irritation to an organism. The carrier usable in the present invention is not particularly limited to the kind thereof, and any carrier may be used as long as the carrier is commonly used in the art and is pharmaceutically acceptable. Non-limiting examples of the carrier may include saline, sterile water, Ringer's solution, buffered physiological saline, an albumin infusion solution, a dextrose solution, a maltodextrin solution, glycerol, ethanol, and the like. These may be used alone or in a mixture of two or more thereof.

In an embodiment of the present invention, it was identified that when the concentration of TGF-β1 exceeds 1046.7 pg/1x10⁵ cells within a culture obtained by subculture of stem cells to P2, the stem cells showed excellent cartilage differentiation capability when subcultured to P6. Therefore, the cell therapy product and pharmaceutical composition manufactured by the methods of the present invention can be advantageously used in the prevention and treatment of arthritis.

### [Mode for Carrying Out the Invention]

Hereinafter, the present invention will be described in detail with reference to examples and experimental examples. However, these examples and experimental examples are given for specifically illustrating the present disclosure, and the scope of the present disclosure is not limited thereto.

### Example 1: Comparative observation of TGF-β1 secretion capability by different lots of early-passage umbilical cord blood-derived mesenchymal stem cells

Umbilical cord blood-derived mesenchymal stem cells were subjected to primary culture, seeded at 2,000-10,000 cells/cm² per passage, and then cultured for 2-6 days, followed by subculturing.

For the comparison of the TGF-β1 secretion capability of early-passage umbilical cord blood-derived mesenchymal stem cells, the umbilical cord blood-derived mesenchymal stem cells from eight lots at passage P2 were analyzed for the concentration of TGF-β1 secreted per 1x10⁵ cells.

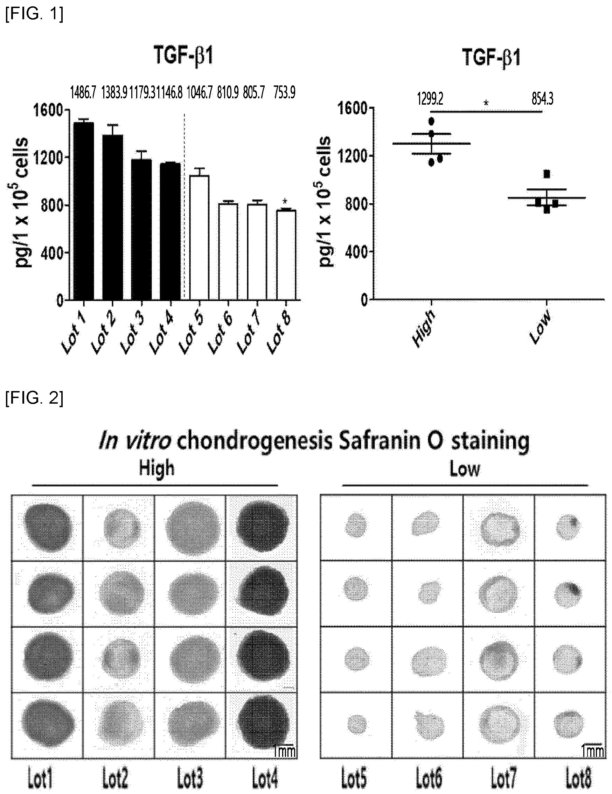

The P2 umbilical cord blood-derived mesenchymal stem cells were cultured in KSB-3 complete media, seeded at 40,000 cells/400 µL in a 48-well plate, and cultured at 37°C in a 5% CO₂ incubator for 96 hours. By the end of culturing, the culture was obtained, and centrifuged at 500 g for 5 minutes to collect the supernatant. The cells were treated with TrypLE^{™} to obtain single cells, which were then subjected to cell counting. The collected supernatant was measured for the concentration of TGF-β1 by using TGF-1 Quantikine ELISA kit. The result values were corrected by the measured cell count and expressed as pg/1x10⁵ cells. All data were analyzed using the Student's T-test and one-way ANOVA. The analysis was performed using GraphPad Prism Software, with p<0.05 or p<0.01 considered statistically significant. The results are shown in Table 1 and FIG. 1.

**TABLE 1**

| | **Lot 1** | **Lot 2** | **Lot 3** | **Lot 4** | **Lot 5** | **Lot 6** | **Lot 7** | **Lot 8** |
|---|---|---|---|---|---|---|---|---|
| Mean | 1486.7 | 1383.9 | 1213.8 | 1146.8 | 1046.7 | 810.9 | 805.7 | 753.9 |
| s.d.(±) | 36.13 | 88.75 | 53.46 | 14.72 | 62.75 | 26.27 | 38.25 | 19.92 |

| | | | | | | | | |
|---|---|---|---|---|---|---|---|---|
| (Unit: pg/1x10⁵ cells) | | | | | | | | |

The analysis results confirmed that the TGF-β1 secretion capability of different lots of umbilical cord blood-derived mesenchymal stem cells was different, ranging from 1486.7 pg/1x10⁵ cells to 753.9 pg/1x10⁵ cells. When the lots were arranged in descending order of TGF-β1 secretion and classified into High and Low groups, the means for the groups were different, 1290.2 pg/1x10⁵ cells and 854.3 pg/1x10⁵ cells, respectively, with the difference being statistically significant.

These results identified that the TGF-β1 secretion capability values by lots of umbilical cord blood-derived mesenchymal stem cells were significantly different. (FIG. 1).

### Example 2: Comparative observation of cartilage differentiation capability of late-passage umbilical cord blood-derived mesenchymal stem cells according to TGF-β1 secretion ability of early-passage umbilical cord blood-derived mesenchymal stem cells

For the comparison of cartilage differentiation capability of late-passage umbilical cord blood-derived mesenchymal stem cells according to TGF-β1 secretion ability of early-passage umbilical cord blood-derived mesenchymal stem cells, eight lots of umbilical cord blood-derived mesenchymal stem cells at passage P6 were analyzed for cartilage differentiation capability.

For the comparison of cartilage differentiation capability, the eight lots of umbilical cord blood-derived mesenchymal stem cells in Example 1 were dispensed at passage P6 into 15 mL tubes at a concentration of 3×10⁵ cells/mL, centrifuged, and then cultured in a 37°C, 5% CO₂ incubator for one day. The pellets thus generated were transferred to a 96-well round-bottom plate, and the medium was replaced with a cartilage differentiation medium (Promocell Mesenchymal Stem Cell Chondrogenic Medium Kit). Subsequently, the pellets were subjected to differentiation induction for three weeks while the medium replacement was conducted three times per week. After completion of the differentiation, the pellets were washed with PBS, fixed with 4% PFA, and then allowed for Safranin-O staining by an analysis service company.

The Safranin-O staining results confirmed that the cartilage differentiation capability values by different lots of umbilical cord blood-derived mesenchymal stem cells were different, and especially, the cartilage differentiation capability of umbilical cord blood-derived mesenchymal stem cells from four lots in the high group classified according to the higher order of TGF-β1 secretion was generally higher than the cartilage differentiation capability in the low group (FIG. 2).

### Example 3: Quantitative evaluation of cartilage differentiation capability by different lots of late-passage umbilical cord blood-derived mesenchymal stem cells

For the more detailed comparative analysis of cartilage differentiation capability, a quantitative evaluation was conducted on the basis of the results of Safranin-O staining indicating cartilage differentiation capability. For the quantitative evaluation, values were obtained by calculating the Safranin-O positive area (%) within the entire pellet area using the color threshold tool of ImageJ software. All data were analyzed using the Student's T-test and one-way ANOVA. The analysis was performed using GraphPad Prism Software, with p<0.05 or p<0.01 considered statistically significant.

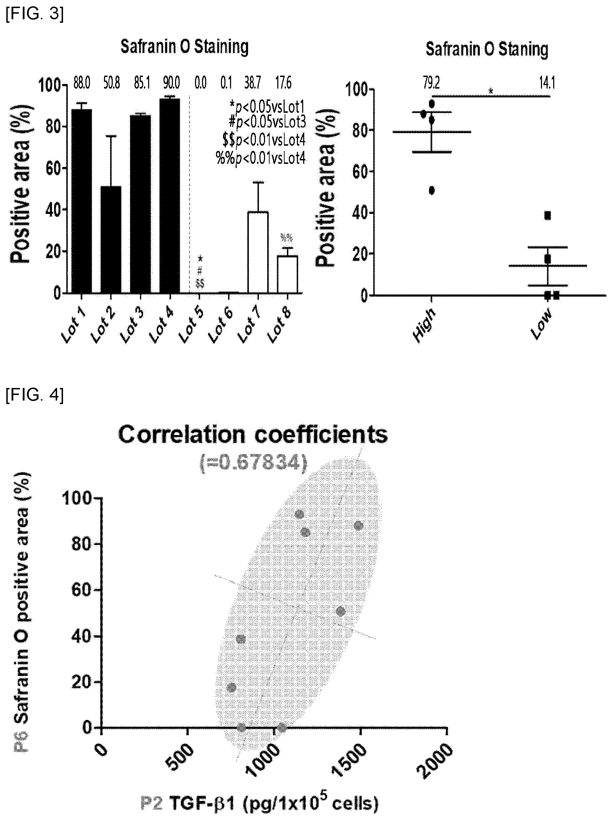

As shown in FIG. 3, the quantitative evaluation results confirmed that the Safranin O positive area values (%) of eight lots varied, ranging from about 90.0% to 0.0%, wherein four lots of the High group in terms of TGF-β1 secretion showed a mean Safranin O positive area value (%) of about 79.2% and the four lots of the Low group showed a mean Safranin O positive area value (%) of about 14.1%, with a significant difference.

It could be therefore identified that the TGF-β1 secretion ability of the early-passage umbilical cord blood-derived mesenchymal stem cells was correlated with the cartilage differentiation capability of the early-passage umbilical cord blood-derived mesenchymal stem cells. Furthermore, on the basis of the results for Lot 4 and Lot 5, the cut-off value of TGF-β1 concentration within a culture of early-passage umbilical cord blood-derived mesenchymal stem cells, which significantly affects the cartilage differentiation capability, could be identified.

### Example 4: Evaluation of correlation coefficient of TGF-β1 secretion capability of early-passage umbilical cord blood-derived mesenchymal stem cells and cartilage differentiation capability of late-passage umbilical cord blood-derived mesenchymal stem cells

To analyze the correlation between TGF-β1 secretion ability of early-passage umbilical cord blood-derived mesenchymal stem cells and the cartilage differentiation capability results of late-passage umbilical cord blood-derived mesenchymal stem cells, correlation coefficients were examined. The results are shown in FIG. 4.

As an analysis results of correlation coefficients between TGF-β1 secretion ability result values at passage P2 and Safranin O positive area (%) result values at passage P6 in the umbilical cord blood-derived mesenchymal stem cells from a total of eight lots, the correlation coefficient was 0.67934.

It could be therefore identified that the TGF-β1 secretion ability of early-passage umbilical cord blood-derived mesenchymal stem cells had "a positive correlation" with the cartilage differentiation capability of late-passage umbilical cord blood-derived mesenchymal stem cells, and the TGF-β1 secretion ability at early passages enables the prediction of *in vitro* cartilage differentiation capability at late passages.

### Example 5: Non-clinical evaluation of cartilage regeneration efficacy according to TGF-β1 secretion capability of early-passage umbilical cord blood-derived mesenchymal stem cells in arthritis model

For the comparison of cartilage regeneration capability after *in vivo* administration between the High cell group (Lot 1) and the Low cell group (Lot 7) in terms of TGF-β1 secretion capability at the early passage, which were classified from the umbilical cord blood-derived mesenchymal stem cells in Example 1, the cartilage regeneration effect was evaluated.

After arthritis was induced in rabbits through anterior cruciate ligament transection, the umbilical cord blood-derived mesenchymal stem cells with high TGF-β1 secretion ability and the umbilical cord blood-derived mesenchymal stem cells with low TGF-β1 secretion ability were administered in an intra-articular manner at Week 6. At eight weeks after the administration, the knee joint cartilage tissues were collected, fixed with 4% PFA, and then allowed for Safranin-O staining by an analysis service company.

The regeneration efficacy evaluation through Safranin-O staining confirmed that the group administered umbilical cord blood-derived mesenchymal stem cells with relatively high TGF-β1 secretion ability (MSCs(High)) showed a higher degree of cartilage regeneration compared with the group administered umbilical cord blood-derived mesenchymal stem cells with relatively low TGF-β1 secretion ability (MSCs(Low)). Furthermore, the histological result-based quantitative evaluation results through Mankin scores confirmed a similar trend wherein the group administered umbilical cord blood-derived mesenchymal stem cells with relatively high TGF-β1 secretion ability (MSCs(High)) showed a higher regeneration degree compared with the group administered umbilical cord blood-derived mesenchymal stem cells with relatively low TGF-β1 secretion ability (MSCs(Low)).

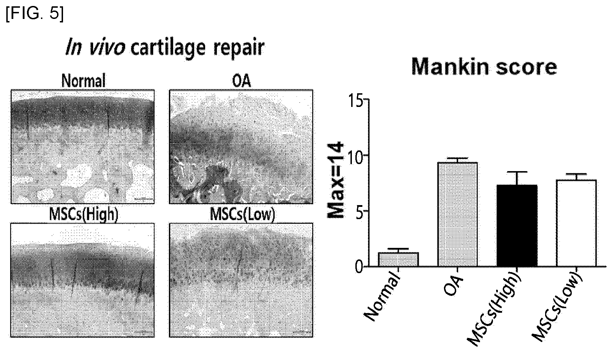

It could be therefore identified that the TGF-β1 secretion ability of early-passage (P2) umbilical cord blood-derived mesenchymal stem cells enables the prediction of *in vitro* cartilage differentiation capability as well as *in vitro* cartilage tissue regeneration capability of late-passage (P6) umbilical cord blood-derived mesenchymal stem cells, and can be utilized as a criterion for selecting cells for arthritis treatment purposes (FIG. 5).

While the present invention has been described with reference to the particular illustrative embodiments, a person skilled in the art to which the present invention pertains can understand that the present invention may be embodied in other specific forms without departing from the technical spirit or essential characteristics thereof. Therefore, the embodiments described above should be construed as being exemplified and not limiting the present invention. The scope of the invention should be construed that the meaning and scope of the appended claims rather than the detailed description and all changes or variations derived from the equivalent concepts fall within the scope of the present invention.

## Claims

1. A method for manufacturing a cell therapy product, the method comprising:
measuring the concentration of TGF-β1 within a culture of stem cells subcultured for 2 passages or fewer; and
isolating stem cells secreting TGF-β1, the concentration of which exceeds 1046.7 pg/1x10⁵ cells within the culture.

2. The method of claim 1, further comprising culturing or proliferating the stem cells secreting TGF-β1, the concentration of which exceeds 1046.7 pg/1x10⁵ cells within a culture of stem cells subcultured for 2 passages or fewer,
wherein the cell therapy product comprises the stem cells secreting TGF-β1, the concentration of which exceeds 1046.7 pg/1x10⁵ cells within a culture of stem cells subcultured for 2 passages or fewer, or stem cells or differentiated cells, which are proliferated or differentiated therefrom.

3. The method of claim 1, wherein the method comprises isolating stem cells secreting TGF-β1, the concentration of which is no less than 1132.12 pg/1x10⁵ cells within a culture of stem cells subcultured for 2 passages or fewer.

4. The method of claim 1, wherein the stem cells are umbilical cord blood-derived mesenchymal stem cells.

5. The method of claim 1, wherein the cell therapy product is a cell therapy product for the prevention or treatment of arthritis.

6. The method of claim 1, wherein the medium is KSB-3 medium.

7. A pharmaceutical composition for the prevention or treatment of arthritis, the pharmaceutical composition comprising, as an active ingredient, stem cells or cells differentiated therefrom,
wherein the cells are stem cells secreting TGF-β1, the concentration of which exceeds 1046.7 pg/1x10⁵ cells within a culture of stem cells subcultured for 2 passages or fewer, or cells proliferated or differentiated therefrom.

8. A method for selecting stem cells for the prevention or treatment of arthritis, the method comprising:
measuring the concentration of TGF-β1 within a culture of stem cells subcultured for 2 passages or fewer; and
selecting, as stem cells for the prevention or treatment of arthritis, stem cells secreting TGF-β1, the concentration of which exceeds 1046.7 pg/1x10⁵ cells within the culture.
